(19) Europäisches Patentamt
European Patent Office
Office européen des brevets

(11) Veröffentlichungsnummer : **0 244 663 B1**

(12) # EUROPÄISCHE PATENTSCHRIFT

(45) Veröffentlichungstag der Patentschrift : **10.07.91 Patentblatt 91/28**

(51) Int. Cl.$^5$ : **A23L 1/015**, A23L 2/34

(21) Anmeldenummer : **87105337.7**

(22) Anmeldetag : **10.04.87**

(54) **Verfahren zur Verminderung des Nitratgehalts von pflanzlichen Lebensmitteln.**

(30) Priorität : **06.05.86 DE 3615321**

(43) Veröffentlichungstag der Anmeldung : **11.11.87 Patentblatt 87/46**

(45) Bekanntmachung des Hinweises auf die Patenterteilung : **10.07.91 Patentblatt 91/28**

(84) Benannte Vertragsstaaten : **AT BE CH DE FR GB IT LI NL SE**

(56) Entgegenhaltungen :
**EP-A- 0 166 090**
**FR-A- 2 057 924**

(56) Entgegenhaltungen :
**FSTA, 84-01-h0006, 84001248; I. NILSSON: "Denitrification of water using immobilized Pseudomonas denitrificans cells", EUROPEAN JOURNAL OF APPLIED MICROBIOLOGY AND BIOTECHNOLOGY, Band 10, Nr. 4, Seiten 268-274, 37 Ref. 1980**
**Schlegel, Allgemeine Mikrobiologie 5. Auflage, 1981**

(73) Patentinhaber : **Eden-Waren GmbH**
**Königsteiner Strasse 107**
**W-6232 Bad Soden (DE)**

(72) Erfinder : **Stetter, Karl Otto, Prof. Dr.**
**Roter Brachweg 2d**
**W-8400 Regensburg (DE)**

(74) Vertreter : **Reitzner, Bruno, Dr. et al**
**Patentanwälte Dipl.-Ing. R. Splanemann Dr. B. Reitzner Tal 13**
**W-8000 München 2 (DE)**

**EP 0 244 663 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Verminderung des Nitratgehalts von pflanzlichen Lebensmitteln.

Bestimmte pflanzliche Lebensmittel, insbesondere Gemüse, können einen hohen natürlichen Nitratgehalt haben, der u.a. von der Pflanzenart, den Düngergaben, denen die Pflanzen ausgesetzt waren, und dem Erntezeitpunkt abhängt. Besonders hohe Nitratgehalte (bis zu 2-5 mg/g erntefrisches Pflanzenmaterial) finden sich bei Rettichen, Roter Bete und Spinat. Der natürliche Nitratgehalt ist etwas niedriger bei Kohl, Möhren und Sellerie.

Dieser Nitratgehalt wird bei dem Verzehr der pflanzlichen Lebensmittel zum Teil durch die natürliche Mundflora und zum anderen Teil im Darm mit Hilfe von Nitratreduzierern, beispielsweise von Kolibakterien sowie anderen Enterobakterien anaerob zu Nitrit abgebaut. Das Nitrit kann mit stickstoffhaltigen organischen Verbindungen zu Nitrosaminen weiter reagieren, denen zumindest zum Teil kanzerogene Wirkungen zugeschrieben werden. Nitrate sind in pflanzlicher Babynahrung besonders unerwünscht, weil sie in Verbindung mit einer Dysbakterie in höherer Konzentration zu der sogenannten Methämoglobinanämie beim Säugling führen können.

Bisher sah man die wirksamste Möglichkeit, den Nitratgehalt von pflanzlichen Lebensmitteln zu vermindern darin, daß man die Nitratdüngerabgaben möglichst niedrig hielt. Dies ist aber nur in engen Grenzen möglich, da die Pflanzen, insbesondere Wurzelgemüse, Nitrat auch bei geringer Konzentration im Boden für die Blüten- und Samenbildung speichern. Bei der Verarbeitung qualitativ hochwertiger pflanzlicher Lebensmittel müssen immer wieder bestimmte Chargen mit hohen Nitratgehalten zurückgewiesen werden.

Alle diese Maßnahmen reichen jedoch nicht aus, um den Nitratgehalt von bestimmten pflanzlichen Lebensmitteln für Diätzwecke, z.B. für Babynahrung, so weit herabzusetzen, daß die in der Diätverordnung vorgeschriebenen Mengen nicht überschritten wurden. Für derartige Lebensmittel konnten bisher bestimmte Pflanzensorten, wie Rote Bete, einzeln überhaupt nicht verwendet werden, d.h. man mußte sie entweder mit Flüssigkeit oder mit anderen zerkleinerten Pflanzenmaterialien verdünnen.

Aus der EP-A-0 166 090 ist ein Verfahren zur Entfernung von Nitrat- und Nitrit-Ionen aus pflanzlichem Material bekannt, wobei das pflanzliche Material bei einer Temperatur von mindestens 70°C mit einem Eisen-(II)-Salz in einem wäßrigen, sauren Medium mit einem pH-Wert von nicht mehr als 3 behandelt wird.

Die DE-A-20 38 258 betrifft ein Verfahren zur Herstellung eines Gärfutterkonzentrats in faserfreier Form, bei dem das Pflanzenmaterial zerkleinert und der Preßsaft nach Zusatz eines Antioxidans und nach Koagulation der Eiweißfraktion mit einem Mikroorganismus versetzt wird, der fähig ist, Stickstoffquellen in Form von Ammoniak und Nitrat zu verwerten. Als Mikroorganismen werden Hefen und Schimmelpilze verwendet, die aus der Kohlenhydratfraktion und den pflanzlichen Säuren als Energiequellen Zellmasse aufbauen. Zur Entfernung des Nitrats müssen sehr große Mengen an Zellmasse erzeugt werden, wodurch sich die Zusammensetzung des Endproduktes wesentlich von der des Ausgangsproduktes unterscheidet.

Die verwendeten Mikroorganismen erzeugen auch Nebenprodukte, wie Alkohol und unerwünschte Geschmacks- und Aromastoffe, so daß das erhaltene Konzentrat zwar als Futtermittel, nicht aber als Lebensmittel geeignet ist.

Nach der DE-A-18 01 295 wird Spinat zur Verhinderung der Nitritbildung mit einer ungiftigen Säure, z.B. Ascorbinsäure oder Zitronensäure versetzt. Durch den säurezusatz wird das Wachstum der nitritbildenden Spontanflora, z.B. von Escherchia coli, unterdrückt. Der Nitratgehalt des Spinats bleibt hierbei unverändert.

In der Literaturstelle "Die Kälte", Sept. 1969, S. 459 bis 474 werden die zeit- und temperaturabhängigen Veränderungen der Keim- und Nitritkonzentration sowie die Mikroflora bei der Lagerung von aufgetautem, hitzebehandeltem Tiefgefrier-Spinatpüree untersucht, wobei auch die Nitratreduktion durch Bakterien im allgemeinen angesprochen wird.

Es fehlt aber jeglicher Hinweis darauf, daß diese Mikroorganismen dazu verwendet werden könnten, um das Nitrat im Spinat vollständig zu Stickstoff abzubauen, da nur der Mechanismus der Nitritbildung untersucht wird.

In der Literaturstelle "International Journal of Food Microbiology" 2 (1985), S. 219 bis 225 ist die Nitratreduktion bei der Fermentation von Karotten bzw. Karottensaft durch gramnegative Bakterien beschrieben.

Es wurden sowohl Bakteriengemische als auch nicht näher definierte Isolate verwendet. Einige dieser Gemische bzw. Isolate bewirkten zwar eine mehr oder weniger starke Nitratreduktion. Die Verfasser weisen aber darauf hin, daß es sich bei diesen Bakterien um Fäulniserreger handelt und daß sich als Nebenprodukte auch gasförmige Stickstoffverbindungen (nitrose Gase) bilden. Daneben ist bekannt, daß einige dieser Bakterien auch störende Aromastoffe und Antibiotika bilden und unerwünschte Farbveränderungen verursachen. Die Verfasser schlagen deshalb als Zukunftsperspektive vor, die Denitrifizierungseigenschaften durch Genmanipulation auf Starterkulturen von Milchsäurebakterien zu übertragen. Genmanipulationen von gramnegativen Bakterien auf grampositive Bakterien, zu denen die Milchsäurebakterien gehören, sind aber mit den heutigen Mitteln der Gentechnologie nur äußerst schwierig und aufwendig durchzuführen.

Aus der Literaturstelle "European Journal of Applied Microbiology and Biotechnology", Vol. 10, No. 4, Seiten 261 bis 274 (1980) (FSTA 84-01-h0 006, 84 001 248) ist ein Verfahren zur Denitrifizierung von Trinkwasser unter Verwendung von immobilisierten Pseudomonas denitrificans-Zellen in Anwesenheit einer exogenen Kohlenstoffquelle bekannt. Bei diesem Verfahren beträgt die Halbwertszeit der Nitratreduzierung bei einem Nitratgehalt von 100 mg $NO_3$/l 30 Tage, was bei Lebensmitteln zu lang ist, da diese aufgrund ihres Gehaltes an organischen Substanzen leicht in Fäulnis übergehen.

Der Erfindung liegt die Aufgabe zugrunde, den Nitratgehalt von pflanzlichen Lebensmitteln, insbesondere von Gemüse, durch dissimilatorischen Nitratabbau auf gesundheitlich unbedenkliche Werte herabzusetzen, ohne daß der Kohlenhydratgehalt des pflanzlichen Lebensmittels nennenswert vermindert wird, ohne daß das pflanzliche Lebensmittel eine Qualitätsminderung, insbesondere im Hinblick auf den Geschmack, das Aroma, die Inhaltsstoffe und die Farbe erleidet, ohne daß Zusätze erforderlich sind.

Diese Aufgabe wird dadurch gelöst, daß man das weitgehend von Fremdkeimen befreite pflanzliche Lebensmittel mit einer Reinkultur oder mit einem definierten Gemisch von ausgewählten denitrifizierenden Bakterien der Arten Paracoccus denitrificans, Pseudomonas stutzeri oder denitrificans oder Bacillus licheniformis versetzt, die in der Lage sind, die Inhaltsstoffe des pflanzlichen Lebensmittels für ihr Wachstum zu nutzen, und zwar ohne wesentliche Verminderung des Gehalts an organischen Inhaltsstoffen und ohne Abgabe geschmacksbeeinträchtigender und farbbeeinflussender Metaboliten und gasförmiger Stickoxide.

Als pflanzliche Lebensmittel, deren Nitratgehalt unter anaeroben Bedingungen mit Hilfe des erfindungsgemäßen Verfahrens vermindert werden kann, kommen insbesondere Rote Beete, Rettiche, Spinat, Möhren oder Kohl in zerkleinerter Form oder in Form von Saft in Frage.

Denitrifizierende Bakterien, einschließlich ihrer Taxonomie sowie Verfahren zu ihrer Isolierung und Züchtung in Reinkulturen sind in der wissenschaftlichen Literatur bereits ausführlich beschrieben. Denitrifzierende Stämme wurden bisher einzeln und in Gruppen gefunden, innerhalb der 22 Bakteriengattungen Acinetobacter, Agrobacterium, Alcaligenes, Aquaspirillum, Bacillus Branhamella, Campylobacter, Corynebacterium, Cytophaga, Gluconobacter, Hyphomicrobium, Kingella, Neisseria, Paracoccus, Propionibacterium, Pseudonomas, Rhizobium, Rhodopseudomonas Spirillum, Thermothrix, Thiobacillus und Thiomicrospira. Diese Bakterien sind als Fäulniserreger ubiquitär in Böden, Gewässern und Kompost verbreitet und spielen eine wichtige Rolle bei der Mineralisierung und im Stickstoffkreislauf der Erde. Sie oxidieren vielfältiges organisches Material, manche aber auch anorganische Stoffe, wie Wasserstoff oder Schwefel. Anaerob sind sie in der Lage, Nitrat oder teilweise auch Nitrit an Stelle von Sauerstoff als Wasserstoffakzeptor zu verwenden, wobei Stickstoff oder $N_2O$ gebildet und Energie gewonnen wird. Manche Stämme bilden auch aus Nitrat Nitrit, ohne letzteres vollständig zu $N_2$ zu reduzieren. Im Unterschied zur Assimilierung von Stickstoff in Zellbausteinen handelt es sich bei der Denitrifizierung um die dissimilatorische Nitratreduktion. Bei einer Verknappung des Angebotes von organischem Substrat sind sie auch in der Lage, interne Reservestoffe (z.B. Glykogen) zu oxidieren, wobei anaerob ebenfalls Nitrat reduziert wird.

Die Erfindung beruht auf der Erkenntnis, daß bestimmte denitrifizierende Bakterien auch in der Lage sind, organische Inhaltsstoffe von pflanzlichen Lebensmitteln für ihr anaerobes Wachstum zu nutzen und hierbei gleichzeitig den Nitratgehalt des pflanzlichen Lebensmittels zu vermindern, wobei nur ein geringer Anteil der reichlich vorhandenen organischen Inhaltsstoffe für das Wachstum benötigt wird, so daß der Charakter des pflanzlichen Lebensmittels praktisch nicht verändert wird, zumal der Anteil der Zellmasse der Bakterien äußerst gering ist.

Diese Erkenntnis war insofern überraschend, als die denitrifizierenden Bakterien in erster Linie als Fäulniserreger wirksam sind, so daß ihr Einsatz zur Behandlung von pflanzlichen Lebensmitteln schlechterdings undenkbar war. Überraschenderweise gibt es aber hierbei Stämme, die die geschmackliche Qualität des Lebensmittels weder durch einen signifikanten Verbrauch der Inhaltsstoffe noch durch störende Metaboliten und gasförmige Stickoxide beeinträchtigen. Wegen des nur geringfügigen Verbrauchs der Inhaltsstoffe braucht man also, um ein Wachstum der denitrifizierenden Bakterien zu erzielen, den Lebensmitteln keine fremden Nährstoffe zuzusetzen. Die ausgewählten denitrifizierenden Bakterien bilden als Metaboliten fast ausschließlich Kohlendioxid, Stickstoff und Wasser. Die Bildung störender Metaboliten, die dem Lebensmittel einen untypischen Geschmack oder eine unerwünschte Färbung verleihen, wird durch Verwendung von Reinkulturen oder von definierten Gemischen von denitrifizierenden Bakterien verhindert, wobei die Auswahl geeigneter Reinkulturen oder definierter Gemische durch Überprüfung des Metabolismus der verwendeten Mikroorganismen vorgenommen wird.

Es ist bekannt, den Nitratgehalt von Wasser zum Zwecke der Trinkwassergewinnung durch Behandlung mit denitrifizierenden Bakterien herabzusetzen. Fin derartigers Verfahren ist z.B. das "Denitropur-Verfahren" der Firma sulzer AG, Winterthur, Schweiz. Bei diesem Verfahren müssen aber dem zu reinigenden Wasser Wasserstoff und Kohlensäure oder organische Nährstoffe, wie Äthanol, Essigsäure oder andere organische Säuren zugesetzt werden. Diese Zusätze sind aber bei der Verarbeitung von pflanzlichen Lebensmitteln nicht

zulässig, da sie einerseits explosionsgefährlich sind und andererseits den Geschmack des Lebensmittels verändern. Dies ist bei dem Verfahren gemäß der Erfindung nicht der Fall, da für das Wachstum der denitrifizierenden Bakterien nur die Inhaltsstoffe des pflanzlichen Lebensmittels genutzt werden.

Für das Wachstum der denitrifizierenden Bakterien wird nur ein sehr geringer Teil dieser Inhaltsstoffe verwendet, so daß sie auch nach einer praktisch vollständigen Entfernung des Nitratgehalts im wesentlichen noch in der ursprüngliche Konzentration vorliegen.

Das pflanzliche Lebensmittel, das zweckmäßig in zerkleinerter Form, z.B. in Form von Schnitzeln oder als Maische, oder aber auch in Form eines Preßsaftes vorliegt, wird zur Durchführung des erfindungsgemäßen Verfahrens in üblicher Weise von Fremdkeimen, insbesondere von Hefe, Schimmelpilzen und Fäulnisbakterien, befreit. Üblicherweise wird es zu diesem Zweck pasteurisiert oder sterilisiert. Auch eine Behandlung mit energiereichen Strahlen ist an sich möglich, obwohl diese Entkeimungsmethode aus lebensmittelrechtlichen Gründen im allgemeinen nicht bevorzugt wird.

Nach der Entkeimung werden die Reinkultur oder das definierte Gemisch von denitrifizierenden Bakterien üblicherweise mit einer Keimzahl von etwa $10^6$ bis $10^7$/ml zugesetzt ; das Wachstum der Bakterien ist gewöhnlich nach Erreichen einer Endkeimzahl von etwa $1 \times 10^8$ bis $5 \times 10^8$/ml, entsprechend einem Bakterien-Trockengewicht von etwa 10 bis 50 µg/ml beendet, da das Nitrat verbraucht ist. Die Anfangs- und Endkeimzahl kann aber je nach der verwendeten Bakterienkultur, dem gewünschten Grad der Verminderung des Nitratgehalts, dem zu behandelnden Produkt und den Reaktionsbedingungen in weiten Grenzen variiert werden. Die Behandlung wird unter anaeroben Bedingungen durchgeführt. Die denitrifizierenden Bakterien können im Endprodukt verbleiben oder aber auch abzentrifugiert werden, wenn man beispielsweise einen klaren Gemüsesaft wünscht.

Nach einer Abwandlung des erfindungsgemäßen Verfahrens kann man dem pflanzlichen Lebensmittel eine Reinkultur oder ein definiertes Gemisch von denitrifizierenden Bakterien in Form einer sich nicht mehr oder nur geringfügig vermehrenden Bakterienmasse der Arten Paracoccus denitrificans, Pseudomonas stutzeri oder denitrificans oder Bacillus licheniformis zusetzen. Eine solche Bakterienmasse verbraucht im wesentlichen nur Nitrat und nur sehr geringe Mengen an zelliniternen Speicherstoffen. In diesem Fall bleiben die sonst für das Wachstum der Bakterien verbrauchten Inhaltsstoffe des pflanzlichen Lebensmittels vollständig erhalten. Da die Bakterien in diesem Fall nicht mehr wachsen, entspricht die zugesetzte Menge der Bakterienmasse gewöhnlich einer Keimzahl von etwa $1 \times 10^8$ bis $5 \times 10^9$/ml, entsprechend entsprechend einem Bakterien-Trockengewicht von etwa 10 bis 500 µg/ml.

Bei beiden Varianten des erfindungsgemäßen Verfahrens werden die denitrifizierenden Bakterien üblicherweise bei einem pH-Wert von etwa 5 bis 7 zugesetzt, da in diesem Bereich sowohl das Wachstum der Bakterien als auch der Nitratabbau optimal gewährleistet sind.

Die Bildung unerwünschter Metaboliten kann auch dadurch verhindert werden, daß man bei dem Verfahren zur Verminderung des Nitratgehalts von pflanzlichen Lebensmitteln unter anaeroben Bedingungen das weitgehend von Fremdkeimen befreite pflanzliche Lebensmittel mit einer Reinkultur oder mit einem definierten Gemisch von ausgewählten denitrifizierenden Bakterien versetzt, die in der Lage sind, die Inhaltsstoffe des Lebensmittels für ihr Wachstum zu nutzen, und zwar ohne wesentliche Verminderung des Gehalts an organischen Inhaltsstoffen und ohne Abgabe von geschmacksbeeinträchtigenden und farbbeeinflussenden Metaboliten und gasförmiger Stickoxide, und die nach folgendem Selektionsprozeß erhältlich sind :

a) Zerkleinerung eines nitrathaltigen oder mit Nitrat versetzten, natürlicherweise Wildstämme von denitrifizierenden Bakterien enthaltenden pflanzlichen Lebensmittels ;

b) Anreicherung der denitrifizierenden Bakterien unter anaeroben Bedingungen ;

c) Auswahl der gasbildenden Proben ;

d) Analyse dieser Proben auf Nitrat und Auswahl der Proben, deren Nitratgehalt signifikant abgenommen hat ;

e) Gewinnung von Einzelkolonien auf sterilisiertem und verfestigtem nitrathaltigem Pflanzensaft oder zerkleinertem Pflanzenmaterial ;

f) Einbringen der Einzelkolonien in sterilen, nitrathaltigen Pflanzensaft und Auswahl der Kolonien nach Gasbildung und Verminderung des Nitratgehalts ;

g) weitere Auswahl durch Bestimmung der Farbabweichung, durch sensorische Prüfung und/oder durch toxikologische Prüfung.

Bei den nach dieser Variante erhältlichen Reinkulturen oder definierten Gemischen handelt es sich auch um solche, die nicht unbedingt den Arten Paracoccus denitrificans, Pseudomonas stutzeri oder denitrificans oder Bacillus licheniformis angehören müssen.

Das für den Selektionsprozeß verwendete Pflanzenmaterial enthält die Wildstämme der denitrifizierenden Bakterien in anhaftenden Bodenteilchen, so daß das Pflanzenmaterial vor der Zerkleinerung zweckmäßig nicht gewaschen wird. Das Pflanzenmaterial wird vorzugsweise zu einer Maische zerkleinert und in einzelne Proben

unterteilt. Die Bakterien wachsen zunächst unter anaeroben Bedingungen, wobei eine Gasbildung auftritt. Bei der Verwertung der Inhaltsstoffe des pflanzlichen Materials durch die Bakterien bildet sich in erster Linie Kohlendioxid, beim Abbau des Nitrats Stickstoff nach folgender Gleichung :

$$2NO_3^- + 10\ [H] + 2H^+ \longrightarrow N_2 + 6H_2O,$$

wobei [H] allgemein einen wasserstoffhaltigen organischen Inhaltsstoff symbolisieren soll. Aus dieser Gleichung ergibt sich, daß der pH-Wert des Reaktionsmediums zunimmt, da $H^+$-Ionen verbraucht werden.
Zur Weiterzüchtung werden die gasbildenden Proben, d.h. die Proben, in denen das Wachstum der denitrifizierenden Bakterien am stärksten ist, ausgewählt. Von diesen Proben werden wiederum diejenigen ausgewählt, bei denen der Nitratgehalt signifikant abgenommen hat. Die Bestimmung des Nitratgehalts kann auf übliche Weise erfolgen, z.B. mit Hilfe von Nitrat-Teststäbchen, durch Hochdruck-Flüssig-Chromatographie und mit Hilfe von Gries'schem Reagens.

Aus den Proben mit dem geringsten Nitratgehalt werden einzelne Mikroorganismen entnommen, die zur Gewinnung von Einzelkolonien auf sterilisierten und zum Beispiel mit Agar oder Kieselsäure verfestigten Pflanzensaft oder zerkleinertes Pflanzenmaterial übertragen werden. Die Einzelkolonien werden dann in sterilen, nitrathaltigen oder mit Nitrat versetzten Pflanzensaft eingebracht und weitergezüchtet. Es werden diejenigen Kolonien ausgewählt, die die höchste Gasbildung und die stärkste Verminderung des Nitratgehalts zeigen. In einem letzten Selektionsschritt erfolgt die Bestimmung der Farbabweichung, eine sensorische Prüfung und/oder eine toxikologische Prüfung. Die Kolonien, die keine untypische Farbabweichung, z.B. Rotoder Braunfärbung zeigen, die bei der sensorischen Prüfung keinen Fremdgeschmack erkennen lassen und die toxikologisch unbedenklich sind, werden als Vorkultur ausgewählt. Die Vorkultur kann entweder unmittelbar in dem Verfahren eingesetzt werden oder aber auch zu einer Starterkultur weitergezüchtet werden. Zur Gewinnung der Starterkultur wird die Vorkultur in einem von Fremdkeimen befreiten zerkleinerten nitrathaltigen pflanzlichen Lebensmittel oder in einer nitrathaltigen Nährbouillon unter anaeroben Bedingungen gezüchtet, worauf die Kultur direkt oder nach dem Abzentrifugieren als Bakterienkonzentrat zur Beimpfung verwendet wird. Auf diese Weise kann das Nitrat-Reduktionsvermögen der Bakterien verbessert werden, da dieser Prozeß ein adaptiver Vorgang ist, der nur anaerob abläuft. Das erhaltene Konzentrat kann dann bis zur Verwendung im tiefgefrorenen Zustand (z.B. bei −20°C) aufbewahrt werden.

Wie vorstehend ausgeführt, erhöht sich bei der Nitrat-Reduktion der pH-Wert des Reaktionsmediums. Diese Erscheinung kann dazu ausgenützt werden, um das mit den denitrifizierenden Bakterien behandelte pflanzliche Lebensmittel einer Nachgärung mit Milchsäurebakterien zu unterziehen. Bekanntlich wird bei einer milchsauren Vergärung von Pflanzenmaterial der Gärungsprozeß bei niedrigen pH-Werten abgestoppt, da die Milchsäurebakterien in einem stärker sauren Medium nicht mehr wachsen können. Da nun die denitrifizierenden Bakterien den pH-Wert erhöhen, fördern sie das Wachstum der Milchsäurebakterien. Man erhält also eine intensivere Milchsäuregärung des pflanzlichen Lebensmittels mit höherer Ausbeute an Milchsäure.

Diese Weiterbildung des erfindungsgemäßen Verfahrens kann beispielsweise zur Herstellung von nitratarmem Sauerkraut und nitratarmen milchsauren Gemüsesäften (Karottensaft, Rote-Bete-Saft) verwendet werden. Bei der Säuerung durch die Milchsäurebakterien findet teilweise eine Lysierung der denitrifizierenden Bakterien statt, so daß im milchsauren Endprodukt keine lebenden denitrifizierenden Bakterien mehr enthalten sind.

Die Erfindung ist durch die nachstehenden Beispiele in nicht einschränkender Weise erläutert.

Beispiel 1

Herstellung von nitratarmen Rote-Bete-Saft

a) Herstellung einer Reinkultur von "Paracoccus denitrificans"

Von 100 Proben Roter Bete verschiedener Herkunft werden je 2 Rüben, die noch etwas außen anhaftendes Erdreich enthalten, getrennt zerkleinert und mit einer Siehschleuder entsaftet. Je 1 ml der Saftproben dient anschließend als Inokulum für je 10 ml sterilen Rote-Bete-Saft in Reagenzgläsern mit Durham-Röhrchen, der zusätzlich 0,15% $KNO_3$ sowie 1 mg/ml Nystatin, letzteres zur Unterdrückung von Hefe- und Schimmelpilzwachstum, enthält. Die beimpften Röhrchen werden anaerob unter einer Stickstoffatmosphäre bei 37°C bebrütet. Nach 3 Tagen zeigen 72 von 100 Röhrchen Fäulnis und Buttersäuregeruch, wobei der pH-Wert in der Regel von 6 auf 5-4 abgefallen ist. Diese Röhrchen werden eliminiert. 28 Röhrchen weisen eine starke

Gasentwicklung auf, und der pH-Wert ist von 6 auf etwa 7.5 angestiegen. Die Schnellanalyse (Merckoquant 10020) des Nitratgehalts, anfangs etwa 3 g/l, ergibt zwischen 0,3 und 0 g/l. Die mikroskopische Inspektion der 28 Anreicherungskulturen zeigt jeweils eine dominierende Flora von kokkenförmigen, unbeweglichen, gramnegativen Bakterien. Von diesen Anreicherungskulturen werden Verdünnungsreihen bis $10^{-9}$ angelegt und von $10^{6}$-$10^{9}$ jeweils 0,1 ml auf Nähragar in Petrischalen ausplattiert und anaerob unter $N_2$ bei 37°C bebrütet. Als Nähragar dient Rote-Bete-Saft (+ 0,15% $KNO_3$), der mit 1,5% Agar versetzt und anschließend sterilisiert worden ist. Nach 5 Tagen zeigen sich auf den Platten von den Verdünnungen $10^{-6}$ bis $10^{-9}$ weißliche bis cremefarbene glänzende Kolonien, etwa 1 bis 2 mm in Durchmesser. Vier mikroskopisch auf ihre Reinheit geprüfte Kokken- Einzelkolonien pro Probe werden in Rote-Bete-Saft (0,15% $KNO_3$) geimpft und bei 37°C anaerob bebrütet ($N_2$). Nach 2 Tagen ist bei 104 Röhrchen eine Gasbildung festzustellen. 15 davon zeigen Kontamination mit beweglichen Stäbchen und werden nicht weiter bearbeitet. Von den 89 Reinkulturen haben 46 den Rote-Bete-Saft braun verfärbt, so daß diese eliminiert werden. Von den verbleibenden 43 Proben mit schöner roter Farbe des Saftes zeigen 38 einen fremdartigen medizinischen oder muffigen Geruch und Geschmack und werden eliminiert. Die übrigen 5 Kulturen beinhalten kein Nitrat mehr (unter 1 mg/l). 2 Kulturen enthalten jedoch etwa 10 mg $NO_2^-$/l, das sie aus Nitrat gebildet hatten, so daß sie ausgesondert werten. Die verbleibenden 3 Kulturen sind zur Denitrifizierung von Rote-Bete-Saft geeignet, da der Saft auch nachher seinen typischen Geruch und Geschmack zeigt. Die Stämme können auch auf künstlichen Medien aerob (z.B. 0,5% Pepton + 0,3% Fleischextrakt, pH 7) sowie anaerob (z.B. 0,9% Pepton + 0,7% NaCl + 0,15% $KNO_3$, pH 7) gezüchtet werden. Es handelt sich jeweils um gramnegative Kokken, 1,1-1,3 µm im Durchmesser, die einzeln, in Paaren und kurzen Ketten vorkommen. Aufgrund ihrer Gestalt sowie ihrer Fähigkeit zu aerobem und anaerobem Wachstum, wobei anaerob eine Denitrifizierung erfolgt, handelt es sich um Stämme der Art "Paracoccus denitrificans". Von einem Isolat wurde ein Guanin + Cytosin-Gehalt der Desoxyribonukleinsäure von 66,5 Mol% bestimmt, was im Einklang mit dem Literaturwert von "Paracoccus denitrificans" ist ("The Prokaryotes", Vol.1, S. 888, Springer, 1981).

b) Herstellung der Starterkulturen

Die Stämme werden routinemäßig als Schrägagarkulturen (0,5% Pepton + 0,3% Fleischextrakt + 1,5% Agar, pH 7) bei 4°C aufbewahrt und alle 3 Wochen überimpft und aerob bei 37°C 2 Tage bebrütet. Zur Gewinnung einer Starterkultur wird zunächst eine Vorkultur gewonnen, wobei 100 ml anaerobes Kulturmedium (0,9 % Penton + 7 g/l NaCl + 0,15% $KNO_3$, pH 7 oder steriler Rote-Bete-Saft) mit 1 öse Schrägagarkultur beimpft und unter $N_2$-Gas 2 Tage bei 37°C bebrütet wird. Diese anaerobe Adaption am Nitrat ist wichtig, um anschließend eine schnelle Denitrifizierung des Saftes zu erreichen. Bei Verwendung aerober Vorkulturen dauert die Denitrifizierung des Saftes etwa zwei- bis fünfmal länger. Mit der Vorkultur werden 100 ml des gleichen Mediums beimpft und in Standkultur ohne Rührung und Begasung bei 37°C inkubiert. Nach 24 Stunden ist eine Zelldichte von $2 \times 10^{8}$/ml erreicht, und die Kultur kann direkt zur Denitrifizierung verwendet werden, oder sie wird steril abzentrifugiert zur Gewinnung eines Zellkonzentrates. Die Zellen werden hierbei anschließend in steriler Magermilch aufgenommen und bei −20°C eingefroren und gelagert.

c) Denitrifizierung des Rote-Bete-Saftes

10 000 l Rote-Bete-Saft werden über einen Plattenerhitzer sterilisiert (90 s, 105°C) und in einen sterilen Gärtank eingeleitet. Der so sterilisierte Saft wird mit 1% der gemäß (b) erhaltenen Starterkultur (Keimgehalt $2 \times 10^{8}$/ml) beimpft. Der Saft hat einen Nitratgehalt von 1301 mg/Liter. Der beimpfte Saft wird 2 Tage bei 37°C bebrütet, wobei eine starke Gasentwicklung auftritt. Nach Beendigung der Gasentwicklung wird ein Keimgehalt von $4 \times 10^{8}$/ml festgestellt. Der Gesamtzuckergehalt des Saftes hat sich von 105 g/l auf 102 g/l geringfügig verringert. Der Nitratgehalt beträgt unter 1 mg/l (spektralphotometrische Bestimmung nach Reduktion von Nitrat zu Nitrit), der Nitritgehalt weniger als 0,2 mg/l (spektralphotometrisch bestimmt). Aflatoxine ($B_1$, $B_2$, $G_1$, $G_2$ ; Nachweisgrenze 0,5 ppb), Nitrosamine (0,5 ppb Nachweisgrenze) sowie Hemmstoffe (Testorganismen : "B subtilis", pH 6, 7, 8 ; "B. stearothermophilusvar. calidolactis", pH 7) wurden nicht festgestellt. Vom erhaltenen Saft werden die Bakterien mittels eines Separators entfernt. Anschließend wird der Saft im Plattenerhitzer (90 s, 105°C) sterilisiert. Der Saft zeigt die für Rote Bete charakteristische tiefrote Farbe und den charakteristischen Geschmack.

d) Milchsaure Vergärung

Der nach (c) erhaltene nitratfreie Saft (pH-Wert = 6,8) wird einer Milchsäuregärung unterzogen, wobei eine

Mischkultur von "Streptococcus lactis" und "Lactobacillus casei" verwendet wird. Die Vergärung erfolgt 15 Stunden bei 45°C, wobei 9,35 g/l reine L (+) -Milchsäure gebildet und ein End-pH-Wert von 4,0 erreicht wird. Der Saft wird anschließend über einen Plattenerhitzer sterilisiert (90 s, 105°C) und abgefüllt.

Beispiel 2

Herstellung von nitratarmem Rote-Bete-Saft mit einer Bakterienmasse

a) Gewinnung der Bakterienmasse

300 l anaerobes Kulturmedium werden analog zu Beispiel 1 (b) mit "Paracoccus denitrificans" (Stamm DSM 1406 der Deutschen Sammlung für Mikroorganismen) beimpft und 2 Tage bei 37°C bebrütet, bis eine Zelldichte von $5 \times 10^8$/ml erreicht ist. Die Bakterien werden anschließend steril abzentrifugiert. Ausbeute : 90g Feuchtgewicht. Diese Zellmasse kann auch für eine spätere Verwendung bei –20°C gelagert werden.

b) Denitrifizierung des Rote-Bete-Saftes

90 g der unter (a) beschriebenen Zellmasse werden in 1 l sterilen Rote-Bete Saft suspendiert und werden 600 l Rote-Bete-Saft (Nitratgehalt: 2600 mg/l), der über einen Plattenerhitzer sterilisiert worden ist (90 s, 105°C) zugesetzt und bei 32°C inkubiert. Nach 5 Stunden ist der Nitratgehalt auf 10 mg/l abgesunken. Der Gesamtzuckergehalt ist unverändert (112 g/l), da die Bakterien offensichtlich zelleigenes Reservematerial zur Aufrechterhaltung ihrer Lebensvorgänge verwendeten. Aufgrund der kurzen Behandlungszeit ist die Farbe von ausgezeichneter Qualität. Die Bakterien werden über einen Separator abzentrifugiert. Sie können bis zu fünfmal verwendet werden, wobei ihre Gärgeschwindigkeit immer schwächer wird.

Beispiel 3

Denitrifizierung von Spinat-Püree mit "Pseudomonas denitrificans

Der Spinat (Nitratgehalt: 1,1 g/kg Frischgewicht) wird gründlich gewaschen, blanchiert (2 min, 85°C) und püriert. Das Püree wird in einem Röhrenerhitzer sterilisiert (200 s, 70°C) und mit einem Vakuumentlüfter behandelt. Dem entlüfteten Püree (100 kg) wird unter $N_2$ in einem Gärbehälter 1 l anaerobe Starterkultur von "Pseudomonas denitrificans" (hergestellt im anaeroben Kulturmedium; Beispiel 1 b) zugesetzt und bei 37°C inkubiert. Nach einem Tag ist der Nitratgehalt auf 8 mg/kg abgesunken, wobei der Spinat weiterhin seine charakteristische Farbe und seinen Geschmack behalten hat. Der Spinat wird anschließend direkt in Gläser abgefüllt und sterilisiert. Wegen seines geringen Nitratgehalts eignet es sich zur Verwendung als Babynahrung.

Beispiel 4

Denitrifizierung von Karottenmaische

Die Karotten (Möhren) werden gewaschen, geschält und blanchiert (z.B. 10 Minuten, 95°C, je nach Größe). Anschließend werden sie zu einer pumpfähigen Maische zermahlen, entlüftet und im Röhrenerhitzer (200 s, 70°C) sterilisiert. Zur Denitrifizierung werden 1.000 kg Karottenmaische (Nitratgehalt: 0,5 g/kg) mit 100 ml eines Bakterienkonzentrates von "Pseudomonas stutzeri" (Keimgehalt : $5 \times 10^{10}$ ßakt./ml) versetzt, das vorher mit sterilem Karottensaft auf 10 l verdünnt worden ist und bei 37°C inkubiert. Es erfolgt eine kräftige Schaumbildung, die nach 2 Tagen beendet ist. Der Nitratgehalt ist unter 1 mg/kg abgesunken. Die Maische wird anschließend homogenisiert und als nitratarme Babykost verarbeitet.

Beispiel 5

Herstellung von nitratarmen Rettichschnitzeln

Rettiche werden gewaschen, geschält, geraspelt und als pumpfähige Maische sterilisiert (200 sec, 70°C Röhrenerhitzer). Die sterilen Schnitzel (1.28 g Nitrat/kg Frischgewicht) werden in einem 100 l Gärbehälter mit einem Gemisch von 2 verschiedenen Stämmen von "Paracoccus denitrificans" versetzt und bei 37°C denitrifiziert. Die verwiendeten Stämme werden von Rettichen in der unter Beispiel 1 angegebenen Weise isoliert und zeigen im Gegensatz zu anderen Stämmen dieser Art ein gutes Wachstum auf Rettichen. Nach 5 Tagen ist

der Nitratgehalt auf 10 mg/kg abgesunken, und die Schnitzel zierden durch Zusatz einer Mischkultur von "Lactobacillus bavaricus" * vergoren. Nach 2 Tagen ist ein Milchsäuregehalt von 0,9 g/kg erreicht (pH 4,3), und die Schnitzel werden abgefüllt und sterilisiert.

* hergestellt nach DE-B- 24 62 152

**Patentansprüche**

1. Verfahren zur Verminderung des Nitratgehalts von pflanzlichen Lebensmitteln, insbesondere von Gemüse, unter anaeroben Bedingungen dadurch gekennzeichnet, daß man das weitgehend von Fremdkeimen befreite pflanzliche Lebensmittel mit einer Reinkultur oder mit einem definierten Gemisch von ausgewählten denitrifizierenden Bakterien der Arten Paracoccus denitrificans, Pseudomonas stutzeri oder denitrificans oder Bacillus licheniformis versetzt, die in der Lage sind, die Inhaltsstoffe des pflanzlichen Lebensmittels für ihr Wachstum zu nutzen, und zwar ohne wesentliche Verminderung des Gehalts an organischen Inhaltsstoffen und ohne Abgabe geschmacksbeeinträchtigender und farbbeeinflussender Metaboliten und gasförmiger Stickoxide.

2. Verfahren nach Anspruch 1, dadurch gekennzeichnet, daß man als pflanzliche Lebensmittel Rote Bete, Rettiche, Spinat, Möhren, oder Kohl in zerkleinerter Form oder in Form von Saft verwendet.

3. Verfahren nach Anspruch 1 oder 2, dadurch gekennzeichnet, daß man die denitrifizierenden Bakterien mit einer Keimzahl von etwa $10^6$ bis $10^7$ /ml zusetzt und das Wachstum der Bakterien nach Erreichen einer Endkeimzahl von etwa $1 \times 10^8$ bis $5 \times 10^8$ /ml entsprechend einem Bakterien-Trockengewicht von etwa 10 bis 50 µg/ml abbricht.

4. Abwandlung des Verfahrens nach Anspruch 1 oder 2, dadurch gekennzeichnet, daß man dem pflanzlichen Lebensmittel eine Reinkultur oder ein definiertes Gemisch von denitrifizierenden Bakterien der Arten Paracoccus denitrificans, Pseudomonas stutzeri oder denitrificans oder Bacillus licheniformis in Form einer sich nicht mehr oder nur geringfügig vermehrenden und im wesentlichen nur Nitrat oder geringe Mengen Kohlenhydrate verbrauchenden Bakterienmasse miteiner Keimzahl von etwa $1 \times 10^8$ bis $5 \times 10^9$ /ml, entsprechend einem Bakterien-Trockengewicht von etwa 10 bis 500 µg/ml zusetzt.

5. Verfahren nach einem der Ansprüche 1 bis 4, dadurch gekennzeichnet, daß man die denitrifizierenden Bakterien bei einem pH-Wert von 5 bis 7 zusetzt.

6. Verfahren zur Verminderung des Nitratgehalts von pflanzlichen Lebensmitteln unter anaeroben Bedingungen, insbesondere Gemüse, dadurch gekennzeichnet, daß das weitgehend von Fremdkeimen befreite, pflanzliche Lebensmittel mit einer Reinkultur oder mit einem definierten Gemisch von ausgewählten, denitrifizierenden Bakterien versetzt wird, die in der Lage sind, die Inhaltsstoffe des pflanzlichen Lebensmittels für ihr Wachstum zu nutzen, und zwar ohne wesentliche Verminderung des Gehalts an organischen Inhaltsstoffen und ohne Abgabe geschmacksbeeinträchtigender und farbbeeinflussender Metaboliten und gasförmiger Stickoxide und die nach folgendem Selektionsprinzip erhältlich sind :

a) Zerkleinerung eines nitrathaltigen oder mit Nitrat versetzten, natürlicherweise Wildstämme von denitrifizierenden Bakterien enthaltenden pflanzlichen Lebensmittels ;

b) Anreicherung der denitrifizierenden Bakterien unter anaeroben Bedingungen ;

c) Auswahl der gasbildenden Proben ;

d) Analyse dieser Proben auf Nitrat und Auswahl der Proben, deren Nitratgehalt signifikant abgenommen hat ;

e) Gewinnung von Einzelkolonien auf sterilisiertem und verfestigtem nitrathaltigem Pflanzensaft oder zerkleinertem Pflanzenmaterial ;

f) Einbringen der Einzelkolonien in sterilen, nitrathaltigen Pflanzensaft und Auswahl der Kolonien nach Gasbildung und Verminderung des Nitratgehalts ;

g) weitere Auswahl durch Bestimmung der Farbabweichung, durch sensorische Prüfung und/oder durch toxikologische Prüfung.

7. Weiterbildung des Verfahrens nach einem der Ansprüche 1 bis 6, dadurch gekekennzeichnet, daß man das mit den denitrifizierenden Bakterien behandelte pflanzliche Lebensmittel einer Nachgärung mit Milchsäurebakterien unterzieht.

8. Verfahren zur Gewinnung einer Vorkultur von denitrifizierenden Bakterien zur Verwendung bei dem Verfahren nach einem der Ansprüche 1 bis 7, gekennzeichnet, durch folgende Verfahrensschritte :

a) Zerkleinerung eines nitrathaltigen oder mit Nitrat versetzten, natürlicherweise Wildstämme von denitrifizierenden Bakterien enthaltenden pflanzlichen Lebensmittels ;

b) Anreicherung der denitrifizierenden Bakterien unter anaeroben Bedingungen ;

c) Auswahl der gasbildenden Proben ;

d) Analyse dieser Proben auf Nitrat und Auswahl der Proben, deren Nitratgehalt signifikant abgenommen hat ;

e) Gewinnung von Einzelkolonien auf sterilisiertem und verfestigtem nitrathaltigem Pflanzensaft oder zerkleinertem Pflanzenmaterial ;

f) Einbringen der Einzelkolonien in sterilen, nitrathaltigen Pflanzensaft und Auswahl der Kolonien nach Gasbildung und Verminderung des Nitratgehalts ;

g) weitere Auswahl durch Bestimmung der Farbabweichung, durch sensorische Prüfung und/oder durch toxikologische Prüfung.

9. Verfahren zur Gewinnung einer Starterkultur von denitrifizierenden Bakterien zur Verwendung bei dem Verfahren nach einem der Ansprüche 1 bis 7, dadurch gekennzeichnet, daß man eine nach Anspruch 8 gewonnene Vorkultur in einem von Fremdkeimen befreiten zerkleinerten nitrathaltigen Nährbouillon unter anaeroben Bedingungen züchtet und die erhaltene Kultur direkt oder nach dem Abzentrifugieren als Bakterienkonzentrat zur Beimpfung verwendet.

10. Verfahren nach Anspruch 9, dadurch gekennzeichnet, daß man das Bakterienkonzentrat bis zur Verwendung im tiefgefrorenen Zustand aufbewahrt.

## Claims

1. A method of reducing the nitrate content of vegetable foodstuffs, in particular vegetables, under anaerobic conditions, characterised in that the vegetable foodstuff, which has been substantially freed of foreign germs, is treated with a pure culture or with a definitive mixture of selected denitrifying bacteria of the variety Paracoccus denitrificans, Pseudomonas stutzeri or denitrificans or Bacillus licheniformis, which are able to utilise the constituents of the vegetable foodstuffs for their growth, that is without substantially reducing the content of organic constituents and without giving off gaseous nitric oxides and metabolites which impair flavour and affect colour.

2. A method according to Claim 1, characterised in that red beetroot, radishes, spinach, carrots or cabbage in comminuted form or in the form of juice are used as the vegetable foodstuff.

3. A method according to Claim 1 or 2, characterised in that the denitrifying bacteria are added with a germ count of approximately $10^6$ to $10^7$ /ml and the growth of the bacteria is discontinued after reaching an final germ count of approximately $1 \times 10^8$ to $5 \times 10^8$ /ml, corresponding to a bacteria dry weight of approximately 10 to 50 µg/ml.

4. A variant of the method according to Claim 1 or 2, characterised by the addition to the vegetable foodstuff of a pure culture or a definitive mixture of denitrifying bacteria of the variety Paracoccus denitrificans, Pseudomonas stutzeri or denitrificans or Bacillus licheniformis in the form of a bacterial composition, which no longer multiplies or multiplies only slightly, which substantially consumes only nitrate or small amounts of carbohydrates and which has a germ count of approximately $1 \times 10^8$ to $5 \times 10^9$ /ml, corresponding to a bacteria dry weight of approximately 10 to 500 µg/ml.

5. A method according to any one of Claims 1 to 4, characterised in that the denitrifying bacteria are added at a pH value of 5 to 7.

6. A method of reducing the nitrate content of vegetable foodstuffs, under anaerobic conditions, in particular vegetables, characterised in that the vegetable foodstuff, which has been substantially freed of foreign germs, is treated with a pure culture or with a definitive mixture of selected denitrifying bacteria which are able to utilise the constituents of the vegetable foodstuffs for their growth, that is without substantially reducing the content of organic constituents and without giving off gaseous nitric oxides and metabolites which impair flavour and affect colour and which can be obtained in accordance with the following selection principle :

a) comminution of a vegetable foodstuff which contains nitrate or has been treated with nitrate and which naturally contains random strains of denitrifying bacteria ;

b) enrichment of the denitrifying bacteria under anaerobic conditions ;

c) selection of gas-forming specimens ;

d) analysis of these specimens for nitrate and selection of those specimens in which the nitrate content has decreased significantly ;

e) obtaining individual colonies in sterilised and solidified nitrate-containing vegetable juice or comminuted vegetable matter ;

f) introduction of the individual colonies into sterile nitrate-containing vegetable juice and selection of colonies according to gas formation and decrease in the nitrate content ;

g) further selection by determining the variation in colour, by verification using sensors and/or by toxicological examination.

7. A development of the method according to any one of Claims 1 to 6, characterised in that the vegetable foodstuff treated with the denitrifying bacteria is subjected to post-fermentation with lactic bacteria.

8. A method of obtaining a preliminary culture of denitrifying bacteria for use in the method according to any of Claims 1 to 7, characterised by the following method steps :

a) comminution of a vegetable foodstuff which contains nitrate or has been treated with nitrate and which naturally contains random strains of denitrifying bacteria ;

b) enrichment of the denitrifying bacteria under anaerobic conditions ;

c) selection of gas-forming specimens ;

d) analysis of these specimens for nitrate and selection of those specimens in which the nitrate content has decreased significantly ;

e) obtaining individual colonies in sterilised and solidified nitrate-containing vegetable juice or comminuted vegetable matter ;

f) introduction of the individual colonies into sterile nitrate-containing vegetable juice and selection of colonies according to gas formation and decrease in the nitrate content ;

g) further selection by determining the variation in colour by verification using sensors and/or by toxicological examination.

9. A method of obtaining a starter culture of denitrifying bacteria for use in the method according to any one of Claims 1 to 7, characterised in that a preliminary culture obtained in accordance with Claim 8 is cultivated under anaerobic conditions in a comminuted nitrate-containing nutrient broth freed of foreign germs and the resultant culture is used directly or after centrifuging as a bacterial concentrate for inoculation.

10. A method according to Claim 9, characterised in that the bacterial concentrate is stored in a deep-frozen state until it is used.

## Revendications

1. Procédé pour diminuer la teneur en nitrates d'aliments végétaux, en particulier de légumes, dans des conditions anaérobies, caractérisé par le fait qu'on mélange l'aliment végétal largement débarrassé des germes étrangers, avec une culture pure ou avec un mélange défini de bactéries dénitrifiantes sélectionnées des genres Paracoccus denitrificans, Pseudomonas stutzeri ou denitrificans ou Bacillus licheniformis, qui sont en mesure d'utiliser, pour leur croissance, les substances de l'aliment végétal, et ce sans diminution substantielle de la teneur en substances organiques et sans élimination de métabolites altérant le goût et influençant la coloration et d'oxydes d'azote gazeux.

2. Procédé selon la revendication 1, caractérisé par le fait qu'on utilise, comme aliment végétal, la betterave rouge, le radis, l'épinard, les carottes ou le chou, sous une forme broyée ou sous forme de jus.

3. Procédé selon la revendication 1 ou 2, caractérisé par le fait qu'on ajoute les bactéries dénitrifiantes avec une quantité de germes d'environ $10^6$ à $10^7$/ml et qu'on arrête la croissance des bactéries après obtention d'une quantité finale de germes d'environ $1 \times 10^8$ à $5 \times 10^8$/ml correspondant à un poids sec de bactéries d'environ 10 à 50 µg/ml.

4. Variante du procédé selon la revendication 1 ou 2, caractérisée par le fait qu'on ajoute à l'aliment végétal une culture pure ou un mélange défini de bactéries dénitrifiantes des genres Paracoccus denitrificans, Pseudomonas stutzeri ou denitrificans ou Bacillus licheniformis sous la forme d'une masse de bactéries ne se multipliant plus ou ne se multipliant que dans une faible mesure et ne consommant essentiellement que des nitrates ou de faibles quantités d'hydrates de carbone, avec une quantité de germes d'environ $1 \times 10^8$ à $5 \times 10^9$/ml, correspondant à un poids sec de bactéries d'environ 10 à 500 µg/ml.

5. Procédé selon l'une des revendications 1 à 4, caractérisé par le fait qu'on ajoute les bactéries dénitrifiantes à une valeur de pH de 5 à 7.

6. Procédé pour diminuer la teneur en nitrates d'aliments végétaux dans des conditions anaérobies, en particulier de légumes, caractérisé par le fait qu'on mélange l'aliment végétal, largement débarrassé des germes étrangers, avec une culture pure ou avec un mélange défini de bactéries dénitrifiantes, sélectionnées, qui sont en mesure d'utiliser pour leur croissance, les substances de l'aliment végétal, et ce, sans diminution substantielle de la teneur en substances organiques et sans élimination de métabolites altérant le goût et influençant la coloration et d'oxydes d'azote gazeux, et qui peuvent être obtenues selon le principe de sélection suivant :

(a) broyage d'un aliment végétal à teneur en nitrates ou mélangé avec des nitrates, contenant de façon naturelle des souches sauvages de bactéries dénitrifiantes ;

(b) enrichissement en bactéries dénitrifiantes dans des conditions anaérobies ;

(c) sélection des échantillons qui forment un gaz ;

(d) analyse de ces échantillons en ce qui concerne les nitrates et sélection des échantillons dont la teneur en nitrates a diminué de façon significative ;

(e) obtention de colonies individuelles sur le jus de plantes ou le matériel végétal broyé à teneur en nitrates, stérilisé et stabilisé ;

(f) introduction des colonies individuelles dans le jus de plantes à teneur en nitrates, stérile, et sélection des colonies après la formation de gaz et la diminution de la teneur en nitrates ;

(g) nouvelle sélection par détermination de la divergence de couleur, par examen sensoriel et/ou par examen toxicologique.

7. Variante du procédé selon l'une des revendications 1 à 6, caractérisée par le fait qu'on soumet l'aliment végétal traité par les bactéries dénitrifiantes à une fermentation ultérieure avec des bactéries lactiques.

8. Procédé d'obtention d'une préculture de bactéries dénitrifiantes pour l'utilisation dans le procédé tel que défini à l'une des revendications 1 à 7, caractérisé par les étapes de procédé suivantes :

(a) broyage d'un aliment végétal à teneur en nitrates ou mélangé avec des nitrates, contenant de façon naturelle des souches sauvages de bactéries dénitrifiantes ;

(b) enrichissement en bactéries dénitrifiantes dans des conditions anaérobies ;

(c) choix des échantillons qui forment un gaz ;

(d) analyse de ces échantillons en ce qui concerne les nitrates et sélection des échantillons dont la teneur en nitrates a diminué de façon significative ;

(e) obtention de colonies individuelles sur le jus de plantes ou le matériel végétal broyé à teneur en nitrates, stérilisé et stabilisé ;

(f) introduction des colonies individuelles dans le jus de plantes à teneur en nitrates, stérile, et sélection des colonies après la formation de gaz et la diminution de la teneur en nitrates ;

(g) nouvelle sélection par détermination de la divergence de couleur, par examen sensoriel et/ou par examen toxicologique.

9. Procédé d'obtention d'une culture de démarrage de bactéries dénitrifiantes pour l'utilisation dans le procédé tel que défini à l'une des revendications 1 à 7, caractérisé par le fait qu'on cultive une préculture obtenue selon la revendication 8 dans un bouillon nutritif à teneur en nitrate, broyé, débarrassé des germes étrangers, dans des conditions anaérobies, et qu'on utilise la culture obtenue pour l'inoculation, directement ou après centrifugation,sous la forme de concentré de bactéries.

10. Procédé selon la revendication 9, caractérisé par le fait qu'on conserve le concentré de bactéries jusqu'à utilisation à l'état surgelé.